# EUROPÄISCHE PATENTSCHRIFT

(11) **EP 2 102 632 B1**
(45) Veröffentlichungstag und Bekanntmachung des Hinweises auf die Patenterteilung: **09.11.2011**
(21) Anmeldenummer: 07856108.1
(22) Anmeldetag: 15.12.2007
(51) Int. Cl.: G01N 21/35, B60T 17/22, G01N 33/28

(54) **ONLINE-SENSOR ZUM ÜBERWACHEN CHEMISCHER VERUNREINIGUNGEN IN HYDRAULISCHEN FLÜSSIGKEITEN**
ONLINE SENSOR FOR MONITORING CHEMICAL CONTAMINATIONS IN HYDRAULIC FLUIDS
CAPTEUR EN LIGNE POUR SURVEILLER LA PRÉSENCE D'IMPURETÉS CHIMIQUES DANS DES LIQUIDES HYDRAULIQUES

(30) Priorität: 18.12.2006 DE 102006060138
(43) Veröffentlichungstag der Anmeldung: 23.09.2009
(73) Patentinhaber: Airbus Opérations SAS, 31060 Toulouse (FR)
(72) Erfinder: CROS, François, 31500 Toulouse (FR); FICKER, Wilhelm, 85604 Pöring (DE); HERTENS, Delphine, 31770 COLOMIERS (FR); KRENKOW, Angelika, 81679 München (DE); LEGNER, Wolfgang, 85635 Höhenkirchen-Siegertsbrunn (DE); MÜLLER, Gerhard, 85567 Grafing (DE); ZIEMANN, Thomas, 84416 Inning am Holz (DE); VAN DEN BOSSCHE, Dominique, 31370 Monès (FR)
(74) Vertreter: Schäfer, Matthias W.
(86) Internationale Anmeldenummer: PCT/DE2007/002258
(87) Internationale Veröffentlichungsnummer: WO 2008/074303

(56) Entgegenhaltungen:
- EP-A- 1 054 251
- EP-A1- 0 989 400
- WO-A-97/14951
- WO-A-02/057774
- WO-A-2004/079366
- WO-A1-99/36772
- WO-A1-02/086035
- DE-A1- 3 816 314
- GB-A- 2 139 763
- US-A- 3 539 804
- US-A- 3 860 344
- US-A- 4 499 376
- US-A- 4 637 729
- US-A- 5 239 860
- US-A- 5 569 842
- US-A- 5 739 916
- US-A1- 2002 118 364
- US-A1- 2008 218 998

## Beschreibung

Die vorliegende Erfindung betrifft einen Online-Sensor zum Überwachen chemischer Verunreinigungen in hydraulischen Flüssigkeiten gemäß dem Oberbegriff des Anspruchs 1.

Hydraulische Fluide für die Luftfahrt sind in der Regel hygroskopisch. Daraus ergibt sich, dass deren Lebensdauer in hohem Maße unvorhersehbar ist. Da die gesamte Hydraulik eines Luftfahrzeugs durch den Zustand des Hydraulikfluids beeinflusst wird, hat die unbemerkte Degeneration des Hydraulikfluids schwerwiegende Folgen, die von Beschädigung bis zum Totalverlust reichen können. Die bisher in der Luftfahrt angewandten Verfahren zur Bestimmung des Zustands des Hydraulikfluids im Hydrauliksystem eines Luftfahrzeugs sind mühsam, zeitaufwändig und teuer. Daher wird das Hydraulikfluid in der Regel nicht häufiger als einmal im Jahr untersucht. Dies birgt ein hohes Risiko mit erheblichen Kosten, wenn die Lebensdauer des Hydraulikfluids unplanmäßig eintritt und dadurch der Flugbetrieb unterbrochen werden muss.

Derzeit ist es üblich, dass die Untersuchung der Hydraulikflüssigkeit "off line", d.h. nach Probenentnahme in einem Labor erfolgt. Hierfür muss Hydraulikflüssigkeit aus dem System am Wartungsstützpunkt abgezapft werden und in ein für derartige Analysen spezialisiertes Labor geschickt werden. Wartungsarbeiten können dann erst nach mehreren Tagen Wartezeit erfolgen, nachdem das Ergebnis vom Labor zurückgekommen ist.

Die interessierenden Parameter des Hydraulikfluids sind dabei insbesondere der Säuregehalt, da dieser kritische Parameter die Lebensdauer definiert. Insbesondere wird durch zu hohen Säuregehalt die Korrosion des Hydrauliksystems, d.h. von Pumpen, Ventilen und Rohrleitungen, befördert. Der Säuregehalt wird mit der Neutralisationszahl TAN bezeichneten. Ferner ist in dem Hydraulikfluid gelöstes Wasser ein wichtiger Parameter, der die Lebensdauer durch Hydrolyse reduziert. Außerdem kann freies Wasser die Pumpen aufgrund mangelnder Schmierung zerstören und gefrieren, was ein Blockieren zur Folge haben kann. Ein weiterer wichtiger Parameter sind die in dem Hydraulikfluid gelösten Gase, die im Falle eines Druckabfalls im System Blasen bilden können und aufgrund von Kavitation zu einem Verlust der Übertragungskraft des Hydraulikfluids führen. Noch ein entscheidender Parameter ist der Chlorgehalt, da Chlorlösungen zur Korrosion von Systemkomponenten des Hydrauliksystems führen können. Außerdem können hierdurch unerwünschte elektrochemische Reaktionen auftreten. Schließlich sind die elektrischen Eigenschaften, d.h. die elektrische Leitfähigkeit und der elektrische Widerstand Parameter, welche die vielfachen Veränderungen des Hydraulikfluids widerspiegeln.

Die Bedeutung dieser Parameter rührt daher, dass Phosphat-Ester, wie sie in Hydraulikfluids für die Luftfahrt vorkommen, polar sind und daher dazu neigen Wasser zu absorbieren. Gelöstes Wasser wiederum kann die Disintegration von Phosphat-Ester Molekülen zur Folge haben, welche entlang dreier Reaktionswege erfolgt: Oxidation, Pyrolyse und Hydrolyse. Die Additive bilden nach der folgenden Gleichung schwache Säuren:

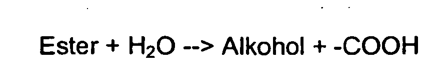

Die Phosphat-Ester bilden nach der folgenden Gleichung starke Säuren:

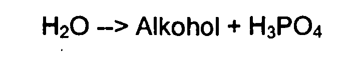

Die Erzeugung von Alkohol kann letztendlich zur Entstehung von Blasen führen, welche die Kraftübertragungseigenschaften des Hydraulikfluids beeinträchtigen können. Andererseits können Phosphorsäure Moleküle mit gelöstem Wasser reagieren und H₃O⁺ Ionen erzeugen, welche Korrosion induzieren.

Aus den oben genannten Gründen ist die Online Überwachung und Beobachtung der Veränderung der relevanten Parameter eines Hydraulikfluids für Luftfahrzeuge von großer Bedeutung.

Aus dem Stand der Technik sind Überwachungssensoren zur Beobachtung der Veränderung des Zustands von Hydraulikfluids bekannt. So beschreibt die US 5,071,527 einen Sensor, der Elektroden zur Messung der elektrischen Eigenschaften einer Probe der zu beobachtenden Hydraulikflüssigkeit aufweist. Dieser Sensor ist mit einer Auswerteeinheit verbunden, welche die Ergebnisse der elektrischen Leitfähigkeitsmessung bestimmten Zuständen des Hydraulikfluids zuordnet. Dabei ist die Sensoreinheit derart klein, dass sie sowohl off-line als auch online eingesetzt werden kann. Die Widerstandsmessung alleine liefert allerdings nur ungenaue und insgesamt unbefriedigende Ergebnisse, so dass hier auf zusätzliche Laboruntersuchungen zurückgegriffen werden muss.

Ferner beschreibt die US 4,013,953 einen optischen Sensor zur Überwachung des Zustandes von Hydraulikflüssigkeiten dessen Messung insbesondere auf der Abschwächung und Streuung des durch eine Probe des zu überwachenden Hydraulikfluids hindurchgeschickten sichtbaren Lichtstrahls beruht. Die hier beschriebene Sensoreinheit ist sehr aufwendig konstruiert und aufgrund der darin enthaltenen beweglichen Teile selbst sehr wartungsanfällig. Da die beschriebene Sensoreinheit ein Geweicht von etwa 1 kg aufweist, wird hier vor allem der off-line Einsatz im Vordergrund stehen.

US 5,739,916 beschreibt eine Vorrichtung, welche den Grad der Kontaminierung von hydraulischen Flüssigkeiten durch Wasser und Ethylenglykol bestimmen kann.

Es ist daher Aufgabe der vorliegenden Erfindung, einen Sensor zur Verfügung zu stellen, der in der Lage ist, die wartungsrelevanten Parameter von Phosphat-Ester basierten hydraulischen Fluiden online zu ermitteln, d.h. ohne diese aus dem Flugzeughydrauliksystem abzuzapfen und Probe zu entnehmen. Damit soll insbesondere Information über den in der Hydraulikflüssigkeit gelöste Wasseranteil und über die Neutralisationszahl TAN (Total Acid Number) gewonnen werden.

Diese Aufgabe wird durch die Merkmale des Anspruchs 1 gelöst. Vorteilhafte Weiterbildungen und Ausführungsformen der Erfindung sind in den abhängigen Ansprüchen angegeben.

Der erfindungsgemäße Online-Sensor zum Überwachen chemischer Verunreinigungen in hydraulischen Fluiden mit einer Aufnahmeeinheit für das zu überwachende Fluid, welche an zwei gegenüberliegenden Seiten angeordnete Sichtfenster aufweist, ist **dadurch gekennzeichnet, dass** der Sensors einen IR (Infrarot)-Emitter und einen IR-Detektor mit vier Detektorfeldem zur IR-Spektroskopie aufweist, die sich gegenüberliegend an den beiden Sichtfenstern angeordnet sind.

Hierdurch wird ein Sensor zur Verfügung gestellt, der in der Lage ist, die wartungsrelevanten Parameter von Phosphat-Ester basierten hydraulischen Fluiden online zu ermitteln, d.h. ohne diese aus dem Flugzeughydrauliksystem abzuzapfen und Probe zu entnehmen. Insbesondere können mit dem erfindungsgemäßen Online-Sensor Informationen über den in der Hydraulikflüssigkeit gelöste Wasseranteil und über die Neutralisationszahl TAN (Total Acid Number) gewonnen werden.

In Versuchen wurde herausgefunden, dass die Absorption von nicht dispersiven IR-Strahlen beim Durchgang durch Phosphat-Ester basiertes Hydraulikfluid aufgrund der Schwingungen der O-H Moleküle in vorbestimmten IR-Durchlassbändern exakten Aufschluss über den Zustand des Hydraulikfluids geben. So ändert sich die Absorption des IR-Strahls bei einer bestimmten Wellenzahl in definierter Weise, je nachdem ob Verunreinigungen durch Wasser, Alkohol oder Säure vorhanden sind. Auch lässt sich der prozentuale Anteil der Verunreinigung auf diese Weise bestimmen. Ferner lässt sich hierdurch die Neutralisationszahl TAN ermitteln.

Aufgrund der geringen Baugröße und des geringen Gewichts des erfindungsgemäßen Online-Sensors kann die Messung online, d.h. im Hydrauliksystem während des Flugbetriebs des Luftfahrzeugs erfolgen und in beliebigen Zeitintervallen, beispielsweise täglich, wiederholt werden. Anhand der so gewonnenen Daten kann der exakte Zustand des Hydraulikfluids und ein entsprechender Trend bestimmt und Wartungstätigkeiten strategisch, beispielsweise zusammen mit anderen vorgesehenen Wartungstätigkeiten geplant werden.

zwischen dem einen Sichtfenster und dem IR-Detektor des erfindungsgemäßen Online-Sensors ist ein optisches Filter mit vier Feldern für IR-Durchlassbänder mit unterschiedlichen Wellenzahlen vorgesehen. Hierdurch wird eine rein optisch basierte qualitative und quantitative Bewertung der Messergebnisse möglich.

Eine vorteilhafte Ausführungsform des erfindungsgemäßen Online-Sensors sieht vor, dass die Sichtfenster aus Saphirglas hergestellt sind. Dies ermöglicht einen streuungsfreien Strahlendurchgang durch die Probe des Hydraulikfluids.

Eine vorteilhafte Ausführungsform des erfindungsgemäßen Online-Sensors sieht vor, dass Einrichtungen zur Online-Auswertung der elektrischen Messsignale des IR-Detektors vorgesehen sind. Diese Einrichtungen können eine Recheneinheit und eine Speichereinheit aufweisen.

Eine vorteilhafte Ausführungsform des erfindungsgemäßen Online-Sensors sieht vor, dass in der Einrichtung zur Online-Auswertung eine Korrelation zwischen der IR-Durchlässigkeit bei zumindest zwei vorbestimmten Wellenzahlen und dem Wassergehalt und/oder dem Alkoholgehalt in dem Hydraulikfluid hinterlegt ist. Diese Daten werden vorher in Versuchen ermittelt und in der Speichereinheit der Einrichtung zur Online-Auswertung hinterlegt.

Eine vorteilhafte Ausführungsform des erfindungsgemäßen Online-Sensors sieht vor, dass in der Einrichtung zur Online-Auswertung eine Korrelation zwischen der IR-Durchlässigkeit bei zumindest zwei vorbestimmten Wellenzahlen und der Neutralisationszahl TAN hinterlegt ist. Diese Daten werden vorher in Versuchen ermittelt und in der Speichereinheit der Einrichtung zur Online-Auswertung hinterlegt.

Das optische Filter des erfindungsgemäßen Online-Sensors weist vier Felder für IR-Durchlassbänder mit unterschiedlichen Wellenzahlen auf, wobei ein erstes IR-Durchlassband die Wellenzahl 3400 cm⁻¹, ein zweites IR-Durchlassband die Wellenzahl 3500 cm⁻¹ und ein drittes IR-Durchlassband die Wellenzahl 3600 cm⁻¹ aufweist, und ein viertes IR-Durchlassband als Referenzfeld vorgesehen ist. Diese Durchlassbänder sind besonders zur Feststellung der Asymmetrie der O-H Absorptionsspitzen bei Phosphat-Ester basierten Hydraulikfluiden geeignet.

Eine vorteilhafte Ausführungsform des erfindungsgemäßen Online-Sensors sieht vor, dass Einrichtungen zum Messen des Lichtdurchgangs im sichtbaren Bereich, vorzugsweise bei 400 nm, vorgesehen sind. Dies verbessert die Aussagekraft der IR-Messung im Bereich starker Oxidation. Dabei ist ein Aufbau mit einem Lichtemitter und einem Lichtdetektor, beispielsweise einer Photodiode, denkbar.

Eine vorteilhafte Ausführungsform des erfindungsgemäßen Online-Sensors sieht vor, dass Einrichtungen zum Messen der Temperatur des Fluids vorgesehen sind. Dabei kann ein entsprechender Temperaturfühler beispielsweise als Thermoelement ausgeführt sein.

Eine vorteilhafte Ausführungsform des erfindungsgemäßen Online-Sensors sieht vor, dass Einrichtungen zum Messen der elektrischen Leitfähigkeit des Fluids vorgesehen sind. Dies kann mittels zweier Elektroden erfolgen. Durch die Messung der Leitfähigkeit können ebenfalls Wasser- und Säuregehalt des Hydraulikfluids bestimmt werden. Dies kann zur Verifizierung der IR-Messergebnisse herangezogen werden.

Weitere, die Erfindung verbessernde Maßnahmen sind in Unteransprüchen angegeben oder werden nachfolgend gemeinsam mit der Beschreibung eines bevorzugten Ausführungsbeispiels der Erfindung anhand der Figuren näher dargestellt. Es zeigen:
- Fig. 1: schematische Ansicht einer vorteilhaften Ausführungsform eines erfindungsgemäßen Online-Sensors;
- Fig. 2: Eine Ansicht des Emitters aus Figur 1 entlang der Linie II - II;
- Fig. 3: Eine Ansicht des Detektors aus Figur 1 entlang der Linie III - III; und
- Fig. 4: Einem Diagramm welches die IR-Durchlässigkeit bei unterschiedlichen Wellenzahlen aufträgt.

Die gezeigten Figuren sind rein beispielhaft schematisch und nicht maßstäblich. Gleiche oder ähnliche Bauteile sind mit gleichen Bezugszeichen versehen. In den Darstellungen wurden die elektrischen und hydraulischen Zu- und Ableitungen aus Gründen der Übersichtlichkeit weggelassen.

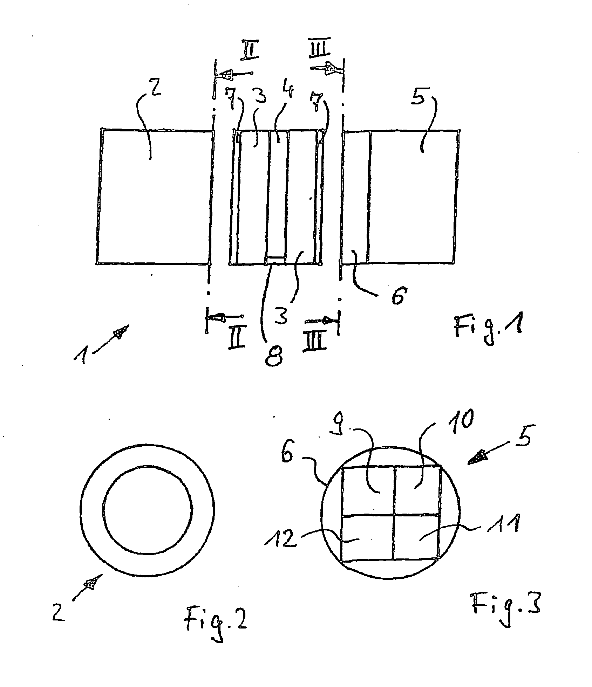

Figur 1 zeigt eine schematische Ansicht einer vorteilhaften Ausführungsform eines erfindungsgemäßen Sensors 1, der im wesentlichen zylinderförmig ausgeführt ist und im wesentlichen drei Baugruppen aufweist. Die mittig angeordnete Baugruppe weist einen Probenbehälter 4 zur Aufnahme einer Probe des zu überwachenden Hydraulikfluids auf. Die Probe umfasst dabei lediglich wenige cm³ des Hydraulikfluids. Es handelt sich bei dem Probenbehälter 4 um eine dünnen scheibenförmigen Aluminiumbehälter, der an den beiden Stirnseiten von Sichtfenstern 3 aus Saphirglas eingefasst ist. An den beiden nach außen gerichteten Seiten der Sichtfenster 3 sind ringförmige Elektroden 7 angeordnet. Ferner ist im unteren Bereich des Probenbehälters 4 ein Temperaturfühler 8 angeordnet, der im vorliegenden Fall als Thermoelement ausgestaltet ist.

In der Zeichnungsebene links von dem Probenbehälter 4 ist ein IR-Emitter 2 angeordnet, der in Figur 2 als Ansicht entlang der Linie II - II in Figur 1 dargestellt ist. Der IR-Emitter 2 ist dabei ein mikrobearbeiteter thermischer IR-Emitter.

In der Zeichnungsebene rechts vom Probenbehälter 4 ist ein zylinderförmiger IR-Detektor 5 mit vier Detektorfeldern dargestellt, wie auch der Ansicht in Figur 3 entnommen werden kann. Der IR-Detektor 5 ist im vorliegenden Ausführungsbeispiel als thermischer Infrarotdetektor ausgestaltet, beispielsweise als Bolometer oder als Thermistor. Aber auch die Verwendung eines speziellen CCD Elements ist denkbar.

Zwischen dem IR-Detektor 5 und dem Probenbehälter 4 ist ein optisches Filter 6 mit vier Feldern mit jeweils unterschiedlichem IR-Durchlassband angeordnet. Die vier Felder des Filters 6 sind im Uhrzeigersinn angeordnet ein Feld 9 als Referenzfeld, ein Feld 10 für die Wellenzahl 3500 cm⁻¹, ein Feld 11 für die Wellenzahl 3600 cm⁻¹ und ein Feld 12 für die Wellenzahl 3400 cm⁻¹ aufweist.

Die Länge des optischen Strahlengangs innerhalb des Hydraulikfluids wird dabei durch den Abstand der beiden infrarotdurchlässigen Sichtfenster 3 bestimmt. Er beträgt im vorliegenden Ausführungsbeispiel 0,3 mm.

Wegen der geringen Größe und des geringen Gewichts des Online-Sensors kann dieser direkt in das Hydrauliksystem eines Luftfahrzeugs, beispielsweise in die Rohrleitungen, integriert werden. Zur Online-Messung, d.h. zur Messung in situ während des Flugbetriebs wird der Infrarotstrahl von dem IR-Emitter 2 durch die Sichtfenster 3 und die im Probenbehälter 4 vorhandene Probe des Hydraulikfluids hindurchgesandt und nach Durchgang durch das Filter 6 mit den vier Durchlassbändern 9,10,11,12 von dem IR-Detektor 5 empfangen. Die Wellenlänge der ausgesandten IR-Strahlung beträgt dabei im vorliegenden Ausführungsbeispiel zwischen 3000 nm und 4000 nm.

Die Messsignale, d.h. die Absorption der IR-Strahlung, werden in dem IR-Detektor gewandelt und als elektrische Signale an eine (nicht gezeigte) Einrichtung zur Online-Auswertung weitergeleitet. Diese Einrichtung weist im wesentliche ein Recheneinheit und einen Datenspeicher auf. Durch Vergleich der aktuellen Messergebnisse mit hinterlegten Daten, kann sofort festgestellt werden, ob sich der Zustand des Hydraulikfluids in einem gesunden Bereich bewegt oder ob ein zu hoher Wasseranteil oder eine Säurebildung vorliegt.

Zusätzlich sind bei dem in Figuren 1 bis 3 gezeigten Online-Sensor 1 noch zwei Elektroden 7 angeordnet, die zu einer Leitfähigkeitsmessung herangebogen werden, um die per IR-Messung ermittelten Werte zu verifizieren. Dabei sind die Elektroden als Platinelektroden ausgeführt und auf ein Keramiksubstrat aufgedruckt. Um Polarisationseffekte zu vermeiden werden die Elektroden mit einer Wechselspannung mit einer Frequenz von 1 kHz beaufschlagt. Auch die Temperaturmessung mit dem Temperaturfühler 8 kann als Verifikation der IR-Messergebnisse dienen und die Funktionstüchtigkeit der IR-Sensoren bestätigen.

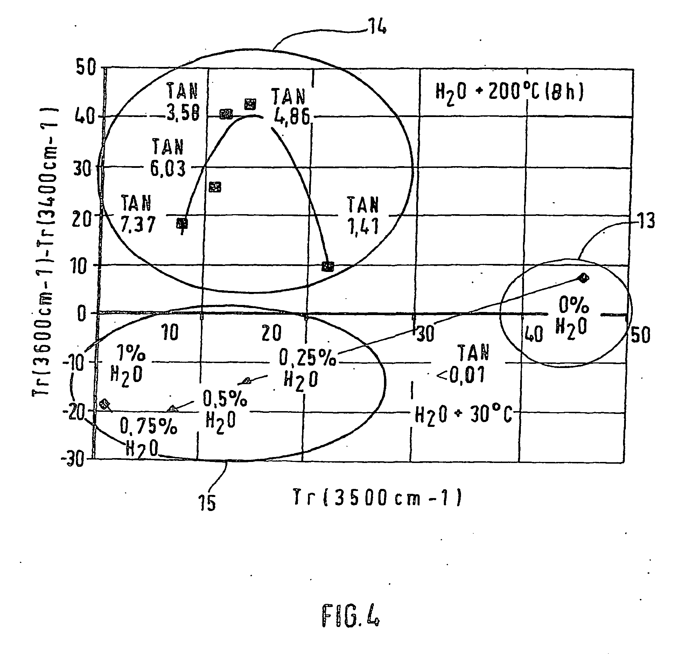

Grafisch lässt sich diese Auswertung der IR-Spektrographie beispielhaft in einem Diagramm gemäß Figur 4 darstellen. Darin ist die IR-Durchlässigkeit in Prozent für das IR-Durchlassband der Wellenzahl 3500 cm⁻¹ auf der Abszisse aufgetragen und mit Tr (3500 cm⁻¹) gekennzeichnet. Auf der Ordinate sind die IR-Durchlässigkeiten in Prozent für die IR-Durchlassbänder der Wellenzahlen 3600 cm⁻¹ und 3400 cm⁻¹ aufgetragen und mit Tr(3600 cm⁻¹) - Tr(3400 cm⁻¹) gekennzeichnet. Die Darstellung trägt den Asymmetrien in den drei unterschiedlichen Durchlassbändern Rechnung und ermöglicht eine eindeutige Festlegung, on der Zustand des Hydraulikfluids in einem "gesunden" Bereich 13, einem "ungesunden" sauren Bereich 14 oder in einem "ungesunden" Bereich mit Wasserabsorption 15 angesiedelt ist.

Die vorliegende Erfindung beschränkt sich in ihrer Ausführung nicht auf das vorstehend angegebene, bevorzugte Ausführungsbeispiel. Vielmehr ist, beschrämht durch der Schutzumfang der anghänglen Ansprüche, eine Anzahl von Varianten denkbar, welche von der dargestellten Lösung auch bei grundsätzlich anders gearteten Ausführungen Gebrauch macht.

### Bezugszeichenliste

- 1: Online-Sensor
- 2: IR Emitter
- 3: Sichtfenster
- 4: Probenbehälter
- 5: IR Detektor
- 6: Optisches Filter
- 7: Elektrode
- 8: Temperaturfühler
- 9: Referenz Filter
- 10: 3500 cm⁻¹ Filter
- 11: 3600 cm⁻¹ Filter
- 12: 3400 cm⁻¹ Filter
- 13: Gesunder Bereich
- 14: Sauerer Bereich
- 15: Bereich mit Wasserabsorption

## Patentansprüche

1. Online-Sensor (1) zum Überwachen chemischer Verunreinigungen einer Phosphat-Ester basierten hydraulischen Flüssigkeit mit einer Aufnahmeeinheit (4) für die zu überwachende hydraulische Flüssigkeit, welche an zwei gegenüberliegenden Seiten angeordnete Sichtfenster (3) aufweist,
**dadurch gekennzeichnet, dass** der Online-Sensor (1) in einem Hydrauliksystem eines Luftfahrzeugs angeordnet ist und einen IR-Emitter (2) und einen IR-Detektor (5) mit vier Detektorfeldem zur IR-Spektroskopie aufweist, die sich gegenüberliegend an den beiden Sichtfenstern (3) angeordnet sind, wobei zur qualitativen und quantitativen Bewertung der Verunreinigungen zwischen dem einen Sichtfenster (3) und dem IR-Detektor (5) ein optisches Filter (6) mit vier Feldern (9,10,11,12) für IR-Durchlassbänder mit unterschiedlichen Wellenzahlen vorgesehen ist, wobei ein erstes IR-Durchlassband die Wellenzahl 3400 cm⁻¹, ein zweites IR-Durchlassband die Wellenzahl 3500 cm⁻¹ und ein drittes IR-Durchlassband die Wellenzahl 3600 cm⁻¹ aufweist und wobei ein viertes IR-Durchlassband als Referenzfeld vorgesehen ist.

2. Online-Sensor (1) nach Anspruch 1,
**dadurch gekennzeichnet, dass** die Sichtfenster (3) aus Saphirglas hergestellt sind.

3. Online-Sensor (1) nach Anspruch 1,
**dadurch gekennzeichnet, dass** Einrichtungen zur Online-Auswertung der elektrischen Messsignale des IR-Detektors (5) vorgesehen sind.

4. Online-Sensor (1) nach Anspruch 3,
**dadurch gekennzeichnet, dass** in der Einrichtung zur Online-Auswertung eine Korrelation zwischen der IR-Durchlässigkeit bei zumindest zwei vorbestimmten Wellenzahlen und dem Wassergehalt und/oder dem Alkoholgehalt in der hydraulischen Flüssigkeit hinterlegt ist.

5. Online-Sensor (1) nach Anspruch 3,
**dadurch gekennzeichnet, dass** in der Einrichtung zur Online-Auswertung eine Korrelation zwischen der IR-Durchlässigkeit bei zumindest zwei vorbestimmten Wellenzahlen und der Neutralisationszahl TAN hinterlegt ist.

6. Online-Sensor (1) nach Anspruch 1,
**dadurch gekennzeichnet, dass** eine Messeinrichtung zum Messen des Lichtdurchgangs im sichtbaren Bereich, vorzugsweise bei 400 nm, vorgesehen sind.

7. Online-Sensor (1) nach Anspruch 1,
**dadurch gekennzeichnet, dass** Einrichtungen (8) zum Messen der Temperatur der hydraulischen Flüssigkeit vorgesehen sind.

8. Online-Sensor nach Anspruch 1,
**dadurch gekennzeichnet, dass** Einrichtungen (7) zum Messen der elektrischen Leitfähigkeit der hydraulischen Flüssigkeit vorgesehen sind.

## Claims

1. An online sensor (1) for monitoring chemical contamination in a hydraulic fluid based on phosphate ester, having a receiving (4) for the hydraulic fluid to be monitored, which comprises viewing windows (3) arranged on two opposite sides,
**characterised in that** the online sensor (1) is arranged in a hydraulic system of an aircraft and comprises an IR emitter (2) and an IR detector (5) having four detector fields for IR spectroscopy, which emitter and detector are arranged on the two viewing windows (3) in a mutually opposed manner, an optical filter (6) having four fields (9, 10, 11, 12) for IR transmission bands with different wave numbers being provided between one viewing window (3) and the IR detector (5) for qualitative and quantitative evaluation of the contaminations, a first IR transmission band having the wave number 3400 cm⁻¹, a second IR transmission band having the wave number 3500 cm⁻¹ and a third IR transmission band having the wave number 3600 cm⁻¹, and a fourth IR transmission band being provided as a reference field.

2. The online sensor (1) according to claim 1,
**characterised in that** the viewing windows (3) are produced from sapphire glass.

3. The online sensor (1) according to claim 1,
**characterised in that** means are provided for online analysis of the electric measuring signals of the IR detector (5).

4. The online sensor (1) according to claim 3, **characterised in that** a correlation between the IR transmission at least at Lwo predetermined wave numbers and the water content and/or the alcohol content in the hydraulic fluid is stored in the means for online analysis.

5. The online sensor (1) according to claim 3,
**characterised in that** a correlation between the IR transmission at least at two predetermined wave numbers and the neutralisation number TAN is stored in the means for online analysis.

6. The online sensor (1) according to claim 1,
**characterised in that** a measuring means for measuring light transmission in the visible range, preferably at 400 nm is provided.

7. The online sensor (1) according to claim 1,
**characterised in that** means (8) for measuring the temperature of the hydraulic fluid are provided.

8. The online sensor according to claim 1,
**characterised in that** means (7) for measuring the electrical conductivity of the hydraulic fluid are provided.

## Revendications

1. Un capteur en ligne (1) pour surveiller la présence d'impuretés chimiques dans des fluides hydrauliques à base d'ester de phosphate comprenant une unité réceptrice (4) pour le fluide hydraulique à surveiller, laquelle présente des regards (3) disposés sur deux côtés opposés
**caractérisé en ce que** le capteur en ligne (1) est disposé dans un système hydraulique d'un avion et présente un émetteur infrarouge (2) et un détecteur infrarouge (5) avec quatre champs de détection pour la spectroscopie infrarouge, qui sont disposés en vis-à-vis sur les deux regards (3) et on, pour l'évaluation qualitative et quantitative des impuretés entre l'un des regards (3) et le détecteur infrarouge (5), un filtre optique (6) muni de quatre champs (9, 10, 11, 12) est prévu pour des bandes passantes infrarouges présentant des nombres d'ondes différents, où une première bande passante infrarouge présente un nombre d'ondes de 3400cm⁻¹, une deuxième bande passante infrarouge un nombre d'ondes de 3500cm⁻¹ et une troisième bande passante infrarouge un nombre d'ondes de 3600cm⁻¹, et où une quatrième bande passante infrarouge est prévue pour servir de champ de référence.

2. Capteur en ligne (1) selon la Revendication 1,
**caractérisé en ce que** les regards (3) sont réalisés en verre de saphir.

3. Capteur en ligne (1) selon la Revendication 1,
**caractérisé en ce que** des dispositifs sont prévus pour l'évaluation en ligne des signaux de mesure électrique du detecteur infrarouge (5).

4. Capteur en ligne (1) selon la Revendication 3,
**caractérisé en ce qu'**une corrélation entre le facteur de transmission infrarouge pour au moins deux nombres d'ondes prédéfinis et la teneur en eau et/ou la teneur en alcool du fluide hydraulique est enregistrée dans le dispositif pour l'évaluation en ligne.

5. Capteur en ligne (1) selon la Revendication 3,
**caractérisé en ce qu'**une corrélation entre le facteur de transmission infrarouge pour au moins deux nombres d'ondes prédéfinis et l'indice de neutralisation TAN est enregistrée dans le dispositif pour l'évaluation en ligne.

6. Capteur en ligne (1) selon la Revendication 1,
**caractérisé en ce qu'**un dispositif de mesure est prévu pour mesurer le passage de la lumière dans le spectre visible, de préférence à 400 nm.

7. Capteur en ligne (1) selon la Revendication 1,
**caractérisé en ce que** des dispositifs (8) sont prévus pour mesurer la température du fluide hydraulique.

8. Capteur en ligne (1) selon la Revendication 1,
**caractérisé en ce que** des dispositifs (7) sont prévus pour mesurer la conductivité électrique du fluide hydraulique.
